# EUROPEAN PATENT APPLICATION

(11) **EP 4 464 286 A1**
(43) Date of publication of application: **20.11.2024**
(21) Application number: 23173958.2
(22) Date of filing: 17.05.2023
(51) Int. Cl.: A61F 5/01, A61F 5/048

(54) **ORTHOSIS**

(71) Applicant: Ostbayerische Technische Hochschule Regensburg, 93049 Regensburg (DE)
(72) Inventor: Böhm, Valter, 93049 Regensburg (DE); Schaeffer, Leon, 93133 Burglengenfeld (DE); Dendorfer, Sebastian, 93164 Laaber (DE); Schratzenstaller, Thomas, 93053 Regensburg (DE)
(74) Representative: Lucke, Andreas

(57) **Abstract**

An orthosis is adapted to be fixed to a human body at a fixing position. The orthosis comprises a first fixation element for being fixed to the human body at a first position different from the fixing position and a first connecting system. The first connecting system is adapted to enable a translationally and/or rotationally movable connection of the first fixation element to the orthosis. The first connecting system comprises a first linear retaining element, a second linear retaining element, and a third linear retaining element, each for providing a force along a direction along which the respective linear retaining element extends. The orthosis is adapted for attaching each of the first, second, and third linear retaining element to the first fixation element and to a rest of the orthosis, such that the first, second, and third linear retaining element exerts a respective first, second, and third force onto the first fixation element. The first force and the second force comprise respective components along a first direction with opposite orientations, and comprise respective components along a second direction with a same, first orientation. The third force comprises a component along the second direction with a second orientation opposite to the first orientation.

## Description

### TECHNICAL FIELD

The disclosure relates to an orthosis, and in particular to an orthosis with a tensegrity structure.

### BACKGROUND

Orthoses are used to support body parts, especially extremities and joints, and to correct joint positions. A distinction is made between non-movable and movable orthoses. Non-movable orthoses, called static orthoses, are used to fix and immobilize body parts. Movable orthoses, known as dynamic orthoses, are used in particular to enable or ensure defined mobility with a targeted restriction of the range of motion, to improve and train mobility in the case of muscle weakness and functional impairments of the affected body regions.

A variety of static and dynamic orthoses are known.

From the utility model specification DE 20 2015 008 342 U1, a hand orthosis is known which has a separate forearm brace and a metacarpal brace, which are connected to each other via an elastic, flat but rigid element. The braces, which are adapted to the body parts, are fastened with Velcro straps. When the wrist is deflected from its intended predetermined rest position, a restoring force is built up. The leaf spring is attached by means of elongated holes, so that only a very limited uniaxial movement of the wrist is possible.

According to patent application WO 1997/029717 A1, a knee orthosis is known which has a thigh cuff and a lower leg cuff connected by two identical modified hinge joints arranged concave to each other. These hinge joints allow rotation of the knee joint about a defined major axis of rotation. A hinge joint is composed of three separate plates. An outer plate has three rivets arranged in a triangle. A middle plate contains three grooves into which the three rivets can be inserted. The shape of the grooves allows rotation of the three rivets in accordance with the anatomical axis of rotation of the knee joint. The concavity of the hinge joints in relation to each other allows only a very limited rotation of the knee joint around the secondary and tertiary axes of rotation.

The patent specification US 11,253,385 B1 describes a knee orthosis consisting of four individual rigid metal splints, two on each side. Pairs of the metal splints are hinged together via a hinge joint. Sides of the splints are connected to each other via fixation elements for fixation to a body area. Several pulleys are attached to the front and back of the splints, over which a front and back elastic band is guided, respectively. Deflection of the knee along the main direction of movement is possible within the range of motion of the hinge joint. Lateral deflection is not possible due to the design of the orthosis. The elastic bands counteract the movement of the knee with restoring forces.

A hand orthosis that has a semi-rigid splint or bar on the underside that holds the hand in the neutral position and prevents movement of the wrist is described in US 9,925,083 B2. The splint can be adjusted in terms of the contact pressure applied by means of a pulley system on the upper side. The orthosis is composed of a main splint body which is form-fitted to a human body. On one side, it has a slit, the both sides of which are connected via a roller and lacing system in such a way that the width of the slit and thus the contact pressure of the splint can be varied over the length of the lacing. The splint fully encloses the forearm and wrist area and, in part, the palm area.

The patent specification EP 3 102 161 B1 describes a knee orthosis consisting of a composite of textile or elastic carrier materials, which differ in their modulus of elasticity and direction of orientation. The orthosis encloses the knee and, in a wide area, the upper and lower leg. Due to the different parameters of the textile elements of the orthosis, forces of different magnitudes are exerted on the knee.

A one-piece rigid hand orthosis consisting of a forearm area, a wrist area, and a curved palm area that has a cavity for receiving tools is described in US 7,837,641 B2. Fixation means of the orthosis completely enclose a human wrist.

Patent US 7,553,289 B2 describes a knee orthosis that allows movement of the knee about a principal axis of rotation. The orthosis is composed of upper and lower support assemblies for attachment to the human body, and two torsion spring assemblies that provide articulated connection between the two support assemblies. Each of the torsion spring assemblies includes a torsion spring element and an elastically compressible core element. As the knee moves along the main deflection direction, restoring torques are applied by the torsion spring elements, which are counteracted by the core elements for damping. Movement of the knee joint in other directions is not possible due to the design of the orthosis.

US 10, 357, 390 B2 describes a foot- and lower-leg-orthosis consisting of two separate orthotic components, one of which is applied in a form-fitting manner to the foot and the other to the lower leg over a large area. The two parts are hinged together by means of a classic swivel joint in the area of the ankle. The joint allows one main direction of movement of the foot. By means of a cable, which connects both orthosis components to each other on the back of the foot or lower leg, and an adjustment device, the maximum deflection of the foot along the main direction of movement can be variably set.

In WO1995004507A1 a hand orthosis is described, which allows the movement of the hand in a restricted range. The range of motion of this orthosis is not adjustable. Almost the entire body region in the area of the wrist is enclosed by the orthosis.

### OVERVIEW

Major disadvantages of the above and other known static and dynamic orthoses are:
- The affected region of the body, such as the wrist area, is enclosed by the orthoses and thus not directly accessible, so that rapid access from the medical or therapeutic side is not possible (without removing the orthoses).
- Due to the functional principle used, the orthoses have a significant weight, as their components are large and heavy. A compact or lightweight orthosis is needed to improve the comfort of wearing.
- Known orthoses only allow for limited adjustment, for example only with respect to a single degree of freedom and/or only to a small extent. Improved possibilities for adjustment are needed to tailor the orthosis to a specific client with a given medical condition. Typically, the medical condition changes in the course of therapy, making an orthosis desirable which can be adjusted by the therapist quickly and without expert knowledge in mechanical engineering. For example, the physical strength of the body parts supported by the orthosis may increase over the course of therapy, and the stiffness of the moving parts of the orthosis should preferably increase accordingly. Ideally, restoring forces for different directions of movement of the human body should be adjustable individually.
- The range of motion allowed by the orthosis is limited and in many cases cannot fully cover the maximum possible anatomically permissible range of motion. An enhanced range of motion may be desirable in some situations.

In view of the technical problems laid out above, there is a need for an improved orthosis and an improved method for adjusting the orthosis. This objective is achieved with an orthosis according to claim 1 and a method according to claim 12. The dependent claims refer to advantageous embodiments.

In a first aspect, an orthosis is adapted to be fixed to a human body at a fixing position. The orthosis comprises a first fixation element for being fixed to the human body at a first position different from the fixing position and a first connecting system. The first connecting system is adapted to enable a translationally and/or rotationally movable connection of the first fixation element to the orthosis. The first connecting system comprises a first linear retaining element, a second linear retaining element, and a third linear retaining element, each for providing a force along a direction along which the respective linear retaining element extends. The orthosis is adapted for attaching each of the first, second, and third linear retaining element to the first fixation element and to a rest of the orthosis, such that the first, second, and third linear retaining element exerts a respective first, second, and third force onto the first fixation element. The first force and the second force comprise respective components along a first direction with opposite orientations, and comprise respective components along a second direction with a same, first orientation. The third force comprises a component along the second direction with a second orientation opposite to the first orientation.

A major advantage of the corresponding orthosis is that the first fixation element may be connected to the rest of the orthosis only by the first connecting system, or by the linear retaining elements, respectively, such that an area between the first fixation element and the rest of the orthosis remains widely accessible for diagnosis and medical treatment, as it remains widely uncovered unlike in the case of conventional connecting means.

The first connecting system with the linear retaining elements is compact and lightweight, reducing the overall weight of the orthosis. The retaining elements may be attached to the other components of the orthosis in a relatively simple manner, e.g., with a lug or a hook, which further reduces the weight.

As a further advantage, the linear retaining elements may be selected according to the therapeutic needs of the patient (e.g., by selecting linear retaining elements with stiffnesses and/or lengths according to a physical condition of the patient) in a straightforward manner, and the linear retaining elements can be exchanged quickly to install the respective linear retaining elements and to adjust the orthosis to the therapeutic needs of the patient. Repeated exchange in the course of a therapy is technically and economically practical.

The linear retaining elements may easily be selected to allow for a significant range of motion. For example, elastomer bands or an elastomer network may be provided as the linear retaining elements and can stretch to a significant maximum dilatation. On the other hand, if a limited range of motion is therapeutically desirable, the linear retaining elements may just as well be selected accordingly.

Each of the first, second, and third linear retaining element may be an elastic element adapted to be mechanically biased with a tensile stress when attached to the first fixation element and to the rest of the orthosis.

Corresponding linear retaining elements are particularly small and lightweight and can be exchanged quickly. They may be provided economically, e.g. in the form of elastomer bands or an elastomer network.

The elastic element may comprise or be made of a polymeric elastomer.

The first connecting system may be arranged in a plane when attached to the orthosis.

The first, second, and third linear retaining element may be arranged in a plane when attached to the orthosis.

The orthosis may further comprise a linking element. The orthosis may be adapted for attaching at least one of the first, second, and third linear retaining element to the first fixation element or to the rest of the orthosis via the linking element. The orthosis may further comprise an adjustable connection between the linking element and the first fixation element or to the rest of the orthosis for adjusting a length or an orientation of said linear retaining element. The adjustable connection may be adapted to provide a locked state wherein a position of the linking element relative to the first fixation element or to the rest of the orthosis that the linking element is connected to is fixed, and an unlocked state wherein said position is moveable.

A corresponding linking element may allow for an especially quick adjustment of the orthosis by changing the position of the linking element. Exchanging the entire set of linear retaining elements to change the stiffness may be avoided. The position of the linking element and/or a transition between the locked state and the unlocked state may be adjustable by using a single tool or even without a tool, for example by using wing nuts or thumb screws which may be opened and closed by hand. Consequently, the therapist may adjust the orthosis on the fly when meeting the patient at an arbitrary location, such as the patient's room in a hospital, without keeping various sets of linear retaining elements.

The linking element provides for a particularly fine or even continuous adjustment of the orthosis. For example, major adjustments to the stiffness may be performed by exchanging the linear retaining elements, and minor adjustments may be made by adjusting the position of the linking element. Hence the orthosis meets the patient's needs both over the entire course of a longer therapy and in terms of quick adjustments on a daily basis for the sake of comfort.

The quick adjustment by using the linking element also improves adjusting the orthosis in feedback with the patient. For example, having just changed the set of linear retaining elements, the therapist can make repeated fine adjustments for optimum comfort using the linking element during a session with the patient.

Alternatively, or in addition to changing the magnitude of the forces, the linking element may also be just to modify the direction of the forces to adjust the orthosis to the therapeutic needs.

At least one or at least two or all three of the first linear retaining element, the second linear retaining element, and the third linear retaining element may be attached to the first fixation element or to the rest of the orthosis via the linking element.

The orthosis may comprise a first linking element with the features described above, and a second linking element with the features described above. The adjustable connection of the first linking element may be between the first linking element and the first fixation element. The adjustable connection of the second linking element may be between the second linking element and the rest of the orthosis. The same retaining element or the same retaining elements may be attached via the first linking element to the first fixation element and via the second linking element to the rest of the orthosis.

The first and the second linking element may in interplay provide full adjustability of the retaining element(s), i.e., with respect to its/their mechanical bias, or stiffness, respectively, and with respect to its/their corresponding force direction.

At least one or all of the adjustable connections of the linking element, the first linking element, and the second linking element may be a hinged connection adapted to provide a rotational adjustment or a translational adjustment between the respective linking element and the element it is connected to.

The first fixation element may comprise a first body abut element, and a first body fixation element. The first body abut element may be adapted to abut a first section of the human body. The first body fixation element may be adapted to fix the first body abut element to the human body. The first body abut element may be mechanically rigid.

The rest of the orthosis may comprise a second body abut element, and a second body fixation element. The second body abut element may be adapted to abut a second section of the human body. The second body fixation element may be adapted to fix the second body abut element to the human body. The second body abut element may be mechanically rigid.

The rest of the orthosis may comprise a second fixation element. The second fixation element may comprise the second body abut element and the second body fixation element.

The first connecting system may further comprise a fourth linear retaining element for providing a force along a direction along which the fourth linear retaining element extends. The orthosis may be adapted for attaching the fourth linear retaining element to the first fixation element and to the rest of the orthosis, such that the fourth linear retaining element exerts a fourth force onto the first fixation element. The fourth force may comprise a component along the second direction along the second orientation. The first, second, third, and fourth force may result in a compressive load onto the first fixation element.

A corresponding arrangement may be used to achieve a particularly compact design of the orthosis. As the linear retaining elements are arranged to cause a compressive load onto the first fixation element, attachment points on the rest of the orthosis for the linear retaining elements may be located near each other, such that the orthosis may overall be compact and consequently lightweight.

The first, second, third, and fourth linear retaining element may be arranged in a plane when attached to the orthosis.

The first fixation element and the rest of the orthosis may comprise attachment elements for attaching the first, second, and third linear retaining element to the first fixation element and to the rest of the orthosis. The attachment elements may be located on the first fixation element and on the rest of the orthosis to determine directions of the first, second, and third force.

The positioning of the attachment elements may be used to determine the directions of the forces according to the therapeutic needs of the patient. The attachment elements may, for example, comprise holes for a screw or for connecting the linear retaining elements directly thereto. Their positioning may be determined in the production process of the first fixation element, for example when the first fixation element is produced by an additive process such as 3D printing. In such embodiments, the additive production process gives the possibility to fabricate the first fixation element according to the therapeutic needs of the patient. Alternatively, or in addition, positioning of the attachment elements such as holes may be determined at a later stage after the production of the first fixation element. For example, the holes or additional holes may be created at the therapist's site according to the needs of a specific patient.

One, several, or all of the attachment element(s) and/or one, several, or all of the linear retaining element(s) may be adapted to enable a rotation of the respective linear retaining element(s) around the respective attachment element(s) when the respective linear retaining element(s) is/are connected to the respective linear retaining element(s).

The attachment elements or at least one of the attachment elements may comprise a first adjustment element adapted for adjusting an orientation and/or a mechanical bias of the first, second, or third linear retaining element when attached to the first fixation element and to the rest of the orthosis. For example, the first adjustment element may provide a detachable connection such as a screw connection between the first fixation element and the respective linear retaining element(s).

The first adjustment element may provide a plurality of attachment points on the first fixation element on the rest of the orthosis for the first, second, or third linear retaining element.

The first, second, or third linear retaining element or at least one of the first, second, or third linear retaining element may comprise a second adjustment element for adjusting a mechanical bias of the first, second, or third linear retaining element when attached to the first fixation element and to the rest of the orthosis.

The second adjustment element may provide a plurality of attachment points for attaching the respective linear retaining element to the first fixation element or to the rest of the orthosis.

The orthosis may further comprise at least one additional linear retaining element. The at least one additional linear retaining element may be adapted to be attached to the first fixation element and to the rest of the orthosis in place of the first, second, or third linear retaining element. The at least one additional linear retaining element may be characterized by a stiffness exceeding a stiffness of said linear retaining element to be replaced or exceeding the stiffness of each of the first, second, or third linear retaining element.

Various sets of linear retaining elements may be provided with different, such as increasingly larger, stiffnesses to cover the needs of the patient over the course of the therapy.

At least two or all of the first, second, or third linear retaining element may be formed as an integral piece.

A corresponding integral piece, which may also be referred to as a network, may be exchanged more easily and quickly by the therapist. A risk of mistakenly confusing the linear retaining elements during assembly of the orthosis is avoided.

The orthosis may further comprise at least one length limiter adapted to limit a maximum length of at least one or all of the first, second, and third linear retaining element.

The at least one length limiter may be adapted to be attached to the first fixation element and to the rest of the orthosis in place of or parallel to the first, second, or third linear retaining element.

The at least one length limiter may comprise a wire or a string adapted to be attached to the first fixation element and to the rest of the orthosis in place of or parallel to the first, second, or third linear retaining element.

This way, the design of the orthosis allows for restricting the movements of the patient in a straightforward manner if desired by the therapist.

The orthosis may further comprise at least one rigid connection element. The orthosis may be adapted for attaching the at least one rigid connection element to the first fixation element and to the rest of the orthosis in place of the first, second, or third linear retaining element to suppress at least one degree of freedom of the translationally and/or rotationally movable connection of the first fixation element to the orthosis.

This way, the design of the orthosis allows for suppressing movements of the patient in a straightforward manner if desired by the therapist.

The orthosis may further comprise a second connecting system with the features of the first connecting system described above.

Said connecting system may be applied in a modular way to implement orthoses with multiple degrees of freedom suitable for complex human joints such as a wrist or an ankle, which has three rotational degrees of freedom and exhibits an effective joint displacement during its movement.

The first connecting system may be adapted to adjust a first restoring force of a translationally movable connection of the first fixation element to the orthosis in a first plane, and the second connecting system may be adapted to adjust a second restoring force of a translationally movable connection of the first fixation element to the orthosis in a second plane perpendicular to the first plane. Alternatively, or in addition, the first connecting system may be adapted to adjust a third restoring force of a rotationally movable connection of the first fixation element to the orthosis around a first axis of rotation, and the second connecting system may be adapted to adjust a fourth restoring force of a rotationally movable connection of the first fixation element to the orthosis around a second axis of rotation perpendicular to the first axis of rotation.

The first connecting system may be adapted to adjust the third restoring force, and the second connecting system may be adapted to adjust the fourth restoring force. The orthosis may further comprise a third connecting system with the features of the first connecting system described above. The third connecting system may be adapted to adjust the third restoring force together with the first connecting system. The first connecting system and the third connecting system may be arranged on opposite sides of the first position.

A corresponding arrangement of a pair of connecting systems on opposite sides of the first position on the human body may result in an orthosis forming a framework around the part of the human body to be treated. Two pairs of connecting systems may be provided in the proximity (e.g., in front of and behind as well as to the left and to the right) of the part of the human body to be treated to define restoring forces of the orthosis according to the different degrees of freedom of the human body part.

The first connecting system may be adapted to enable a translational movement of the first fixation element in a plane parallel to a plane in which the first connecting system extends when connected to the orthosis.

The first connecting system and the second connecting system may be oriented perpendicular to each other when connected to the orthosis.

The first fixation element and/or any other fixation element of the orthosis may be provided with a cushioning towards the human body.

Any further fixation elements, if present, may also be provided with a cushioning towards the human body.

The orthosis may be a hand orthosis, a finger orthosis, a shoulder orthosis, or a wrist orthosis or an ankle orthosis.

The first fixation element and/or any other fixation element of the orthosis and/or the rest of the orthosis may be adapted to provide a form-fit and/or a force-fit connection to the human body. The form-fit and/or the force-fit connection to the human body may be based on a measured anatomy of a human body.

Any further fixation elements, if present, may also be adapted to provide a form-fit and/or a force-fit connection to the human body.

According to a second aspect, a method is provided for adjusting an orthosis. The orthosis is adapted to be fixed to a human body at a fixing position. The orthosis comprises a first fixation element for being fixed to the human body at a first position different from the fixing position and a first connecting system. The first connecting system is adapted to enable a translationally and/or rotationally movable connection of the first fixation element to the orthosis. The first connecting system comprises a first linear retaining element, a second linear retaining element, and a third linear retaining element, each for providing a force along a direction along which the respective linear retaining element extends. The first, second, and third linear retaining element is attached to the first fixation element and to a rest of the orthosis, such that the first, second, and third linear retaining element exerts a respective first, second, and third force onto the first fixation element. The first force and the second force comprise respective components along a first direction with opposite orientations, and comprise respective components along a second direction with a same, first orientation. The third force comprises a component along the second direction with a second orientation opposite to the first orientation. The method comprises adjusting an orientation or a mechanical bias of at least one of the first, second, and third linear retaining element.

The orientation and/or the mechanical bias of the at least one of the first, second, and third linear retaining element may be adjusted such that in a physiologically neutral position of a part of the human body located between the fixing position and the first position, no forces result onto the fixing position and/or onto the first position when the orthosis is fixed to the human body.

The orientation and/or the mechanical bias of the at least one of the first, second, and third linear retaining element may be adjusted such that in a physiologically neutral position of a part of the human body located between the fixing position and the first position, a preselected force results onto the first position and/or onto the first fixation element when the orthosis is fixed to the human body.

The orientation and/or the mechanical bias of the at least one of the first, second, and third linear retaining element may be adjusted such that, when a joint of a human body located between the fixing position and the first position is rotated, no forces result onto the joint.

The adjusting the orientation and/or the mechanical bias of at least one of the first, second, and third linear retaining element may comprise replacing at least one of the first, second, and third linear retaining element with at least one additional linear retaining element. The at least one additional linear retaining element may be adapted to provide a stiffness exceeding a stiffness of said linear retaining element to be replaced or exceeding the stiffness of each of the first, second, or third linear retaining element.

The first fixation element and the rest of the orthosis may comprise attachment elements for attaching the first, second, and third linear retaining element to the first fixation element and to the rest of the orthosis. The attachment elements may be located on the first fixation element and on the rest of the orthosis to determine directions of the first, second, and third force. The attachment elements or at least one of the attachment elements may comprise a first adjustment element adapted for adjusting an orientation and/or a mechanical bias of the first, second, or third linear retaining element when the respective linear retaining element is attached to the first fixation element and to the rest of the orthosis. The adjusting the orientation or the mechanical bias of at least one of the first, second, and third linear retaining element may comprise changing the attachment of the at least one of the first, second, and third linear retaining element to the respective attachment element using the first adjustment element.

The first, second, or third linear retaining elements or at least one of the first, second, or third linear retaining element may comprise a second adjustment element. The adjusting the orientation or the mechanical bias of at least one of the first, second, and third linear retaining element may comprise changing the attachment of the at least one of the first, second, and third linear retaining element to the first fixation element or to the rest of the orthosis using the second adjustment element.

The orthosis may further comprise a linking element and an adjustable connection between the linking element and the first fixation element or the rest of the orthosis. At least one of the first linear retaining element, the second linear retaining element, and the third linear retaining element may be attached to the first fixation element or to the rest of the orthosis via the linking element. The adjusting the orientation or the mechanical bias of at least one of the first, second, and third linear retaining element may comprise adjusting a position of the linking element.

The adjustable connection may be adapted to provide a locked state wherein a position of the linking element relative to the first fixation element or to the rest of the orthosis that the linking element is connected to is fixed, and an unlocked state wherein said position is moveable. The adjusting the position of the linking element may be performed while the adjustable connection is in the unlocked state. The method may further comprise setting the adjustable connection to the locked state.

### BRIEF DESCRIPTION OF THE FIGURES

The techniques of the present disclosure and the advantages associated therewith will be best apparent from a description of exemplary embodiments in accordance with the accompanying drawings, in which:
- Fig. 1: gives a side view of an orthosis according to a first embodiment;
- Fig. 2a: gives a side view of an orthosis according to another embodiment;
- Fig. 2b: gives a side view of an orthosis according to another embodiment;
- Fig. 3a: gives a side view of an orthosis according to another embodiment;
- Fig. 3b: gives a side view of an orthosis according to another embodiment;
- Fig. 4: gives a side view of an orthosis according to another embodiment;
- Fig. 5a: gives a side view of an orthosis according to another embodiment;
- Fig. 5b: gives a side view of an orthosis according to another embodiment;
- Fig. 5c: gives a side view of an integral piece combining linear retaining elements, according to an embodiment;
- Fig. 5d: gives a side view of an integral piece combining linear retaining elements, according to another embodiment;
- Fig. 6a: gives a perspective view of an orthosis according to another embodiment;
- Fig. 6b: gives another perspective view of the orthosis according to the embodiment of Fig. 6a;
- Fig. 6c: gives a side view of the orthosis according to the embodiment of Fig. 6a and Fig. 6b;
- Fig. 6d: gives another side view of the orthosis according to the embodiment of Fig. 6a to Fig. 6c;
- Fig. 7a: gives a side view of an orthosis according to another embodiment;
- Fig. 7b: gives a side view of an orthosis according to another embodiment;
- Fig. 7c: gives a side view of an orthosis according to another embodiment;
- Fig. 7d: gives a side view of an orthosis according to another embodiment;
- Fig. 7e: gives a side view of an orthosis according to another embodiment;
- Fig. 8a: gives side views of orthoses according to other embodiments;
- Fig. 8b: gives side views of orthoses according to other embodiments;
- Fig. 9a: gives a side view of an orthosis according to another embodiment;
- Fig. 9b: gives a side view of an orthosis according to another embodiment;
- Fig. 10: gives side views of orthoses according to other embodiments;
- Fig. 11a: gives a perspective view of an orthosis according to another embodiment;
- Fig. 11b: gives another perspective view of the orthosis according to the embodiment of Fig. 11a;
- Fig. 11c: gives a side view of the orthosis according to the embodiment of Fig. 11a and Fig. 11b; and
- Fig. 11d: gives another side view of the orthosis according to the embodiment of Fig. 11a to Fig. 11c.

### DETAILED DESCRIPTION OF EMBODIMENTS

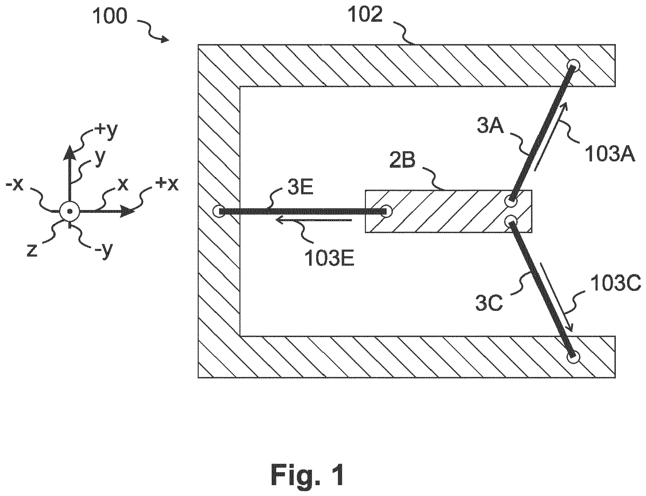

Fig. 1 gives a schematic illustration of an orthosis 100 according to an embodiment.

The orthosis 100 comprises a first fixation element 2B. When the orthosis 100 is worn by a patient, the first fixation element 2B is fixed to the body of the patient at a first position, for example at a hand of the patient.

When the orthosis 100 is worn by the patient, the rest of the orthosis 102 is fixed to the body at a different position, referred to as the fixing position, such that a human joint of the patient that is to be treated is located between the first position and the fixing position. In the example, the rest of the orthosis 102 is fixed to the forearm, such that the wrist of the patient is located between the first position at the hand and the fixing position at the forearm.

A (first) connecting system consisting of three linear retaining elements 3A, 3C, 3E in the form of elastomer bands 3A, 3C, 3E connects the first fixation element 2B to the rest of the orthosis 102. The connecting system allows for a translational and a rotational movement of the first fixation element 2B with respect to the rest of the orthosis 102. When the orthosis 100 is worn by the patient, the patient's hand may thus perform translational and rotational movements with respect to the forearm.

In Fig. 1, the orthosis 100 is shown in an assembled state, meaning that the elastomer bands 3A, 3C, 3E are attached both to the first fixation element 2B and to the rest of the orthosis 102. In an unassembled state, the elastomer bands 3A, 3C, 3E are provided separately from the first fixation element 2B and from the rest of the orthosis 102. In some embodiments, the assembly process is performed at a central production side of the orthosis 100. However, preferably, the elastomer bands 3A, 3C, 3E are provided to a therapist separate from the first fixation element 2B and from the rest of the orthosis 102. The therapist selects the elastomer bands 3A, 3C, 3E according to the patient's need and assembles the orthosis 100 accordingly.

Lengths of the elastomer bands 3A, 3C, 3E as the first, second, and third linear retaining element are such that, when the orthosis 100 is in the assembled state, i.e., the elastomer bands 3A, 3C, 3E are attached to the first fixation element 2B and to the rest of the orthosis 102, the elastomer bands 3A, 3C, 3E are mechanically biased with a tensile stress. Consequently, in the assembled state, the elastomer bands 3A, 3C, 3E exert forces 103A, 103C, 103E onto the first fixation element 2B, respectively. The forces 103A, 103C have different orientations +y, -y along a first direction y, and the same orientation +x along a second direction x. The force 103E has the opposite orientation -x along the second direction x. In an alternative embodiment (not shown), the linear retaining elements are mechanically biased compressively, and, e.g., are provided in the form of springs. In a corresponding embodiment, absolute orientations of all the forces are reversed, without changing their relative (same or opposite) orientations.

Consequently, in the assembled state of the orthosis 100, the first fixation element 2B is held in an equilibrium position defined by the elastomer bands 3A, 3C, 3E and the forces 103A, 103C, 103E those exert, respectively. As a consequence, when the orthosis 100 is worn by the patient, the first position on the patient's body (i.e., the hand in the example) experiences a restoring force when it performs translational and rotational movements. The magnitude and the direction of the force is determined by the arrangement and the selection (i.e., in terms of length and stiffness) of the elastomer bands 3A, 3C, 3E.

The orthosis 100 has a variety of advantages over conventional orthoses: It does not require a conventional bearing (such as a ball bearing), which is large and heavy and therefore reduces the comfort of wearing. Instead, the connecting system is filigree, small and lightweight, thus improving the comfort of wearing. Moreover, it permits access of a therapist to a large region of the patient's body between the first fixation element 2B and the rest of the orthosis 102. The stiffness of the orthosis 100 may, in a straightforward way, be defined or adjusted by selecting elastomer bands 3A, 3C, 3E of proper individual stiffnesses or lengths or by adjusting their mechanical bias. The elastomer bands 3A, 3C, 3E forming the connecting system are economic and may be provided in large sets with different lengths and stiffnesses. Multiple connecting systems may be provided to implement different kinds of orthoses in a modular way according to the degrees of freedom of the body parts or human joints to be treated.

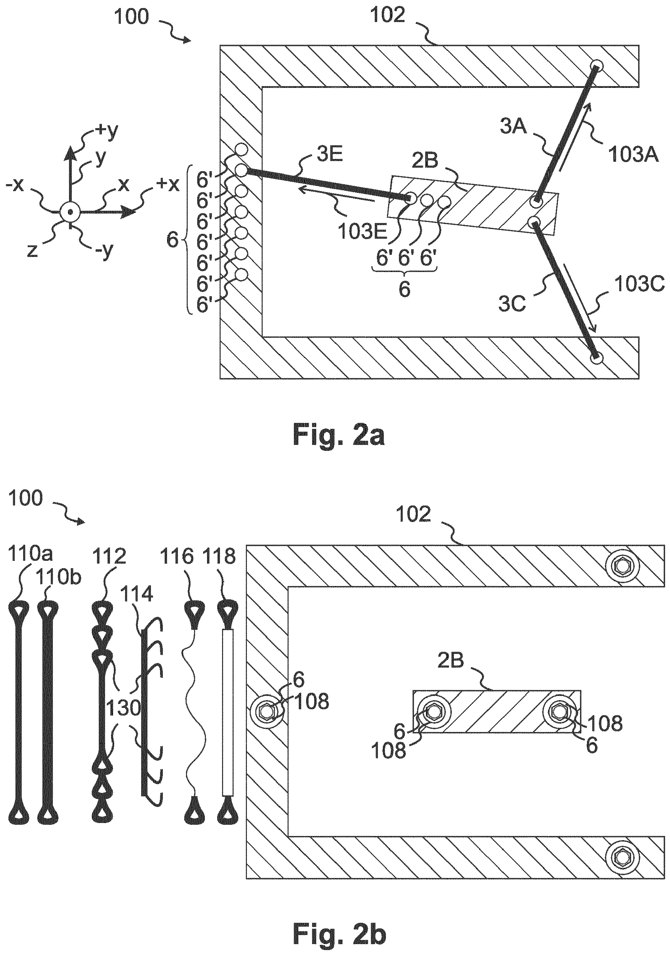

Fig. 2a, Fig. 2b, Fig. 3a, Fig. 3b, and Fig. 4 illustrate modifications of the orthosis 100 to further improve the adjustability. The orthoses 100 of Fig. 2a, Fig. 2b, Fig. 3a, Fig. 3b, and Fig. 4 are similar to the orthosis 100 of Fig. 1. Similar elements are indicated by same reference numerals and for the sake of brevity their description will not be repeated. The modifications of Fig. 2a, Fig. 2b, Fig. 3a, Fig. 3b, and Fig. 4 may be combined to improve the adjustability further. Particularly beneficial are combinations, which combine a modification, which permits to adjust the magnitude of the forces exerted by the linear retaining elements, with a modification, which permits to adjust the direction of the forces exerted by the linear retaining elements. As a result, full adjustability of the forces exerted by the linear retaining elements is achieved.

In the embodiment of Fig. 2a, the linear retaining element 3E is attached to the first fixation element 2B via an attachment element 6 and to the rest of the orthosis 102 via another attachment element 6. The attachment element 6 provides a plurality of attachment points 6' in the form of holes 6'. In alternative embodiments (not shown), hooks 6', or clamping elements 6' are provided as the attachment points 6'. The linear retaining element 3E is selectively attached to one of the holes 6' on the first fixation element 2B and to one of the holes 6' on the rest of the orthosis 102.

In the depicted embodiment, the holes 6' on the first fixation element 2B are arranged such that selecting one of them for attaching the linear retaining element 3E determines the mechanical bias of the linear retaining element 3E, or the magnitude of the corresponding force 103E, respectively. The holes 6' on the rest of the orthosis 102 are arranged such that selecting one of them for attaching the linear retaining element 3E determines the direction of the linear retaining element 3E, or the direction of the corresponding force 103E, respectively. In alternative embodiments (not shown), the holes 6' for determining the magnitude of the force 103E and the holes 6' for determining the direction of the force 103E are both arranged on the first fixation element 2B or are both arranged on the rest of the orthosis 102. In yet other embodiments (not shown) only one of the attachment elements 6 is formed with a plurality of attachment points 6', or holes 6', respectively, and the remaining attachment element 6 only provides one attachment point 6'. In yet other embodiments (not shown), attachment elements 6 with a plurality of attachment points 6', e.g., in the form of holes 6', are provided for several or all linear retaining elements.

In the embodiment of Fig. 2b, the attachment elements 6 are provided in the form of holes 6 which are threaded. Bolts 108 connect to those threaded holes 6. In corresponding embodiments, the linear retaining elements are provided with a lug or a hook at their respective ends. To attach the linear retaining elements to the first fixation element 2B and to the rest of the orthosis 102, the bolts 108 are inserted into the lugs of the linear retaining elements and screwed into the threaded holes 6, or the hooks of the linear retaining elements are hooked to the bolts 108, respectively. In alternative embodiments (not shown), the attachment elements 6 are provided in the form of hooks, clamping elements or other known structures which permit to attach linear retaining elements such as elastomer bands.

The connection between the linear retaining elements and the bolts 108 is formed such that the linear retaining elements can rotate (i.e., with the lugs or hooks at their ends) around the bolts 108. This is achieved by tightening the bolts 108 into the threaded holes 6 only to such degree that the linear retaining elements are still free to rotate (i.e., with their lugs or hooks at their ends) but are not clamped to the first fixation element 2B or to the rest of the orthosis 102.

Fig. 2b also shows different types of linear retaining elements of or for the orthosis 100.

The linear retaining elements 110a, 110b are provided with lugs at their respective ends for attaching them to the orthosis 100 as described above. To adjust the magnitude of the forces exerted by the linear retaining elements, the linear retaining elements 110a, 110b are provided with different thicknesses and stiffnesses, respectively. The magnitude of the forces exerted by the linear retaining elements is adjusted by selecting a specific linear retaining element 110a, 110b and attaching it to the orthosis 100. Alternatively, the linear retaining elements 110a, 110b may be provided with different lengths (not shown) resulting in different mechanical biases when attached to the orthosis 100, which also permits to adjust the magnitude of the forces exerted by selecting a linear retaining element.

The linear retaining element 112 is provided with a second adjustment element 130 for connecting it to the orthosis 100. The second adjustment element 130 consists of several lugs. The magnitude of the force exerted by the linear retaining element 112 is adjusted by selecting one of the lugs for attaching the linear retaining element 112 to the orthosis 100 using the corresponding lug.

The linear retaining element 114 is provided with a second adjustment element 130 for connecting it to the orthosis 100. According to this embodiment, the second adjustment element 130 consists of several hooks. The magnitude of the force exerted by the linear retaining element 114 is adjusted by selecting one of the hooks for attaching the linear retaining element 114 to the orthosis 100 using the corresponding hook.

A length limiter 116 comprises lugs (or, alternatively, hooks (not shown)) for connecting it to the attachment elements 6, and a string or metal wire spanning between the lugs. The string or metal wire has a Young's modulus much larger than the one of the linear retaining elements. In other words, when its ends are pulled apart by the force of a human extremity, it essentially does not extend. The length limiter 116 has a length exceeding the distance between the attachment elements 6 on the first fixation element 2B and on the rest of the orthosis 102 it is to be connected to and is therefore not mechanically biased when attached to the orthosis 100 in place of one of the linear retaining elements 3A, 3C, 3E. The first fixation element 2B connected to the rest of the orthosis 102 via the length limiter 116 can move freely within a range defined by the length of the length limiter 116. When the length limiter 116 is fully extended, the first fixation element 2B reaches the end of this range. As an alternative to replacing a linear retaining element 3A, 3C, 3E, the length limiter 116 is attached to the orthosis 100 in parallel to one of the linear retaining elements 3A, 3C, 3E. For example, such as length limiter can be implemented by integrating a metal wire into an elastomer band of the respective linear retaining element 3A, 3C, 3E (not shown).

A rigid connection element 118 comprises lugs (or, alternatively, hooks (not shown)) for connecting it to the attachment elements 6, and a rigid body spanning between the lugs. The rigid connection element 118 has a length matching the distance between the attachment elements 6 on the first fixation element 2B and on the rest of the orthosis 102 it is to be connected to. One or several rigid connection element(s) 118 can be used to replace one or several linear retaining element(s) 3A, 3C, 3E to implement a static orthosis 100 with respect to the movement of the first fixation element 2B relative to the rest of the orthosis 102.

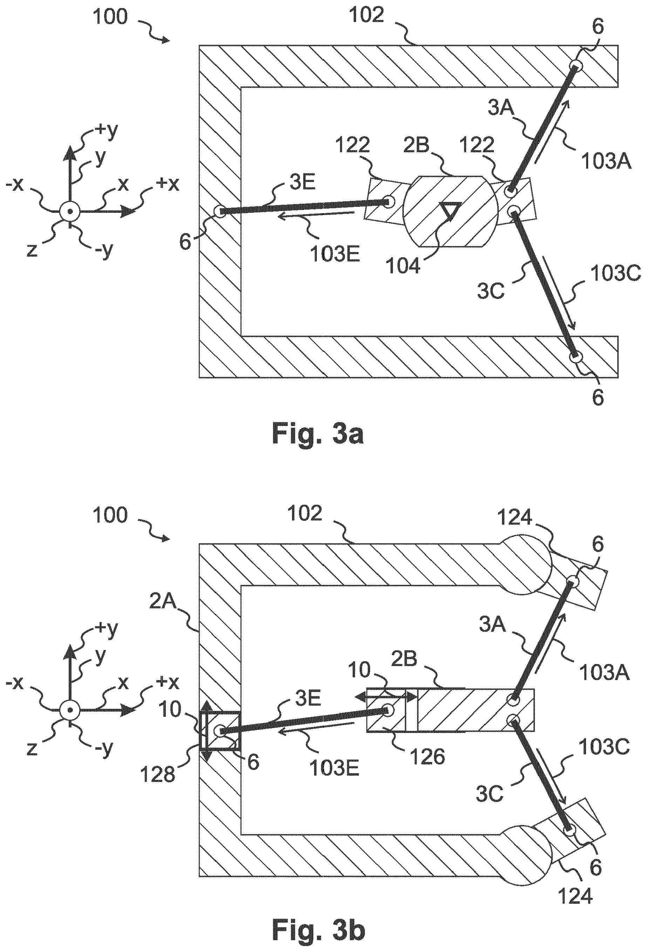

Fig. 3a shows an embodiment of the orthosis 100, wherein the linear retaining elements 3A, 3C, 3E are connected to the first fixation element 2B via linking elements 122.

The linking elements 122 are rigid parts with respective hinged connections to the first fixation element 2B. Attachment elements 6 are provided on the linking elements 122 for attaching the linear retaining elements 3A, 3C, 3E to the first fixation element 2B via the linking elements 122. The linking elements 122 are equipped with a locking mechanism (not shown) in the form of a set screw for selectively blocking the movement of their respective hinged connections to the first fixation element 2B. In some embodiments, the set screw takes the form of a thumb screw which may be tightened and opened without a tool.

In this embodiment, the forces 103A, 103C, 103E are adjusted by adjusting the positions of the linking elements 122, respectively. For this purpose, the set screw of the locking mechanism is loosened, the position of the hinged connection is adjusted to a preselected position, and the set screw is tightened to lock the locking mechanism and hence the hinged connection. This modifies the equilibrium position of the first position 104, at which the orthosis is fixed to the human body, relative to the rest of the orthosis 102.

This procedure can be used to adjust the equilibrium position of the first position 104 to a physiologically neutral position of the part of the human body that the orthosis 100 is connected to at the first position 104. A corresponding adjustment results in a zero net force of the orthosis 100 onto the respective part of the human body when it is in its physiologically neutral position.

Alternatively, the procedure can be used to set a preselected net force of the orthosis 100 onto the respective part of the human body when it is in its physiologically neutral position, both in terms of its direction and its magnitude.

Fig. 3b shows another embodiment of the orthosis 100, wherein the linear retaining element 3E is connected to the first fixation element 2B via a linking element 126. Moreover, the linear retaining elements 3A, 3C, 3E are connected to the rest of the orthosis 102 via linking elements 124, 128.

The linking elements 124 are similar to the linking element 122 on the right-hand side of Fig. 3a. However, the linking elements 124 are provided on the rest of the orthosis 102, whereas the linking element 122 of Fig. 3a is provided on the first fixation element 2B. Both configurations are possible and may be combined where desirable.

The linking elements 126, 128 are similar to the linking element 122 of Fig. 3a. However, the linking elements 126, 128 are connected to the first fixation element 2B, or to the rest of the orthosis 102, respectively, via hinged connections which allows for a translational adjustment 10, whereas the linking element 122 of Fig. 3a provides a rotational connection. Both configurations are possible and may be combined where desirable.

Beneficially, the linking elements 126, 128 together allow for adjusting both the direction and the magnitude of the force 103E via the translational adjustment 10. However, the force may also be fully adjusted using a combining of a linking element with a hinge for translation adjustment and a linking element with a hinge for rotational adjustment, located both on the first fixation element 2B, both on the rest of the orthosis 102, or located on the first fixation element 2B and on the rest of the orthosis 102 (not shown). Alternatively, a linking element may be combined with an attachment element 6 providing a plurality of attachment points 6' to fully adjust the force.

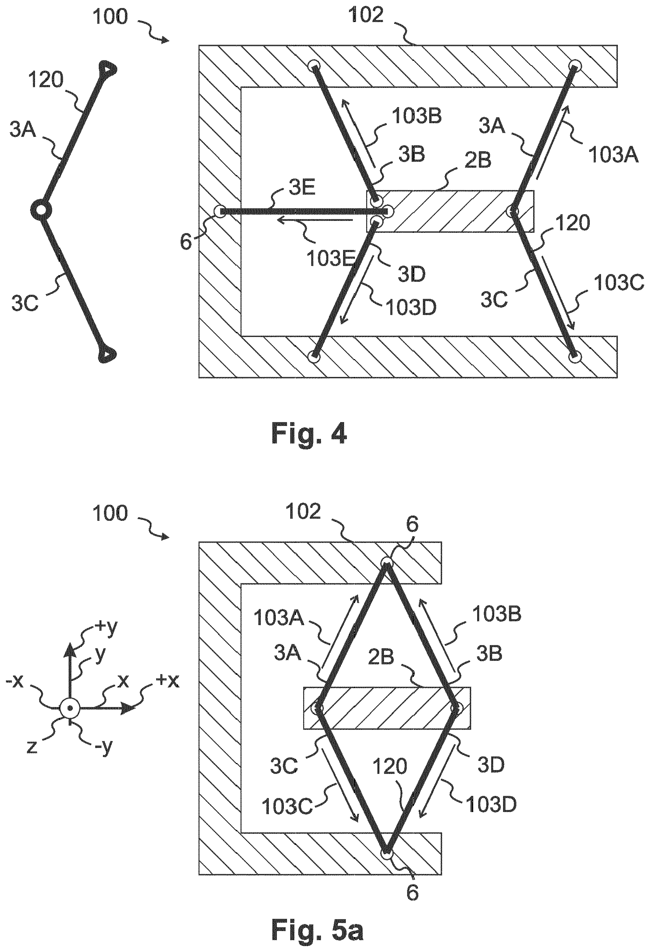

Fig. 4 shows an embodiment wherein the first connecting system comprises additional linear retaining elements 3B, 3D to enable the translationally and rotationally movable connection of the first fixation element 2B to the rest of the orthosis 102.

In the depicted embodiment, the linear retaining element 3E and the additional linear retaining elements 3B, 3D together provide the functionality of the third linear retaining element 3E of the previous embodiments.

As a consequence of this redundancy, the components of the forces 103B, 103D, 103E exerted along the first direction y by the linear retaining elements 3B, 3D, 3E, respectively, can be selected independently of the components of the respective forces 103B, 103D, 103E exerted along the second direction x. For example, the linear retaining elements 3B, 3D can be stiffer than the linear retaining element 3E to implement strong restoring forces along the first direction y. The stiffnesses of the first and second linear retaining elements 3A, 3C are selected according to the stiffness of the linear retaining element 3E to adjust the net restoring force along the second direction x.

Moreover, the additional linear retaining elements 3B, 3D permit to orientate at least one of the linear retaining elements 3A, 3B, 3C, 3D, 3E along a main direction given by the therapeutic treatment to be provided to the patient. For example, the main direction defined by the therapeutic treatment may be the direction along which the forearm of the patient extends, and the linear retaining element 3E may be orientated along this direction. A force or a range of motion along this main direction may be adjusted easily by replacing only the linear retaining element 3E or an associated length limiter 116.

Fig. 4 also shows an integral piece 120 combining the linear retaining elements 3A, 3C. A corresponding integral piece 120 is preferably also provided in any of the other embodiments. Any pair of neighboring linear retaining elements, or any larger set of neighboring linear retaining elements is preferably combined into an integral piece 120. The integral piece 120 may even combine all the linear retaining elements of the corresponding embodiment.

The integral piece 120 combining several linear retaining elements helps to facilitate a quick and simple assembly of the orthosis 100.

Fig. 5a illustrates an embodiment of the orthosis 100 with a fourth linear retaining element 3D attached to the first fixation element 2B and to the rest of the orthosis 102 in addition to the first and second linear retaining elements 3A, 3C and the third linear retaining element 3B.

The linear retaining elements 3A, 3B, 3C, 3D exert respective forces 103A, 103B, 103C, 103D onto the first fixation element 2B. The component of the fourth force 103D along the second direction x has the same orientation -x as the respective component of the third force 103B. The forces 103A, 103B, 103C, 103D result in a compressive load onto the first fixation element 2B along the second direction x, along which the first fixation element 2B extends.

Corresponding embodiments allow for a particularly compact orthosis 100, as the attachment elements 6 on the rest of the orthosis 102 for any pair 3A, 3B; 3C, 3D of neighboring linear retaining elements can be arranged very close to each other. Preferably, the respective attachment elements 6 are combined into a single attachment element 6 as depicted in Fig. 5a.

Orthoses 100 according to embodiments with a compressive load onto the first fixation element 2B along its respective length direction are also referred to as orthoses 100 with a tensegrity structure. A connecting system that gives rise to the compressive load onto a respective fixation element is also referred to as a tensegrity group, or as a two-dimensional tensegrity group.

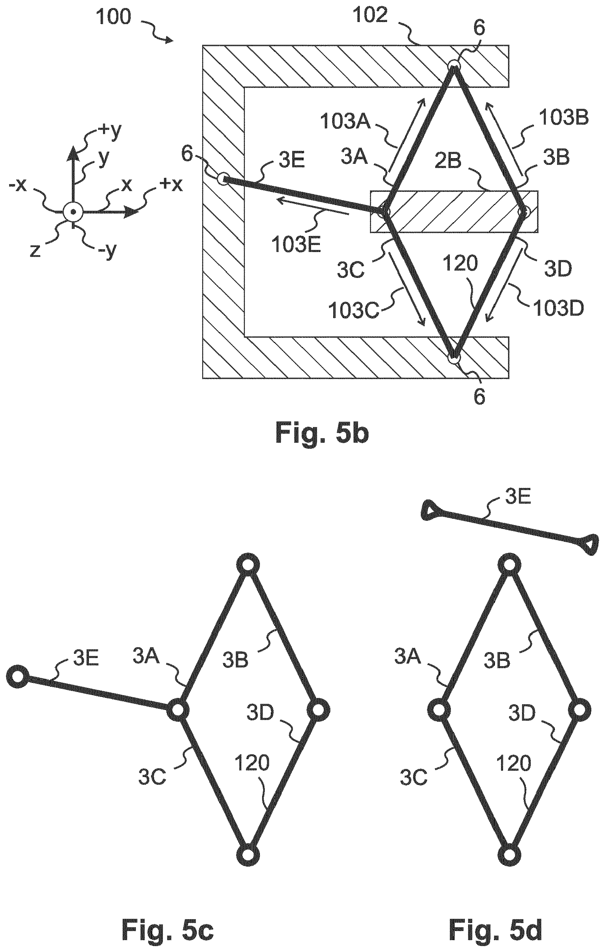

Fig. 5b, Fig. 5c, and Fig. 5d show an embodiment of the orthosis 100 similar to the embodiment of Fig. 5a.

The first connecting system of the orthosis 100 according to the embodiment of Fig. 5b is provided with an additional linear retaining element 3E as compared to the embodiment of Fig. 5a. The additional linear retaining element 3E is orientated along the main direction dictated by the requirements of the therapeutic treatment.

Fig. 5c and Fig. 5d illustrate two possible embodiments of the first connecting system of the orthosis 100 of Fig. 5b.

In the embodiment of Fig. 5c, all linear retaining elements of the connecting system are formed as an integral piece 120. An orthosis 100 with a corresponding connecting system may be assembled particularly quickly and easily.

In the embodiment of Fig. 5d, the first, second, third, and fourth linear retaining elements 3A, 3B, 3C, 3D are formed as an integral piece 120, but the additional linear retaining element 3E is provided as a separate piece. An orthosis 100 with a corresponding connecting system allows for a particularly simple and fast adjustment of the force exerted along the main direction of the therapeutic treatment.

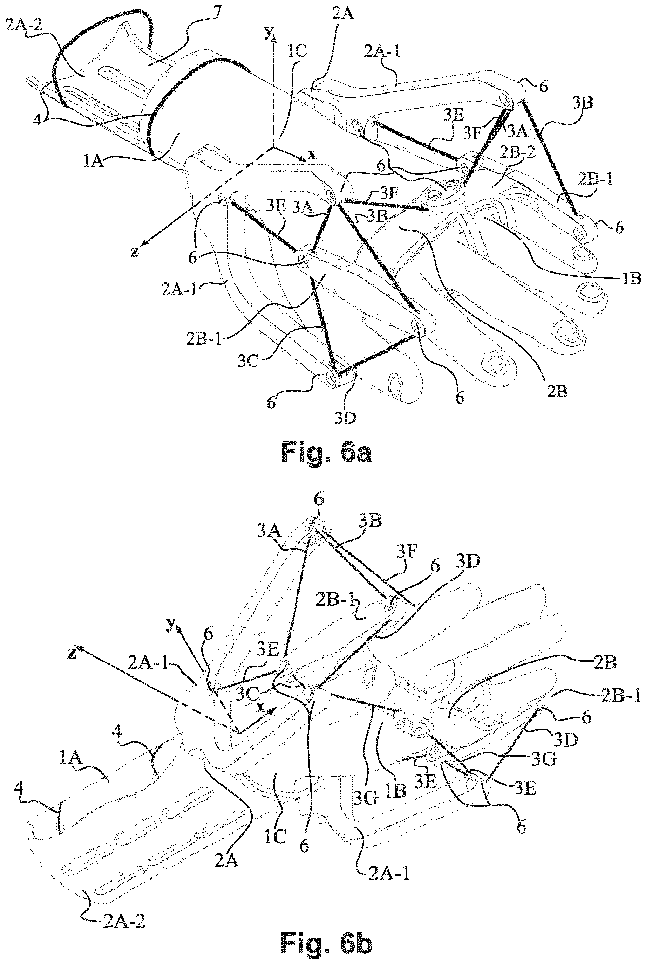

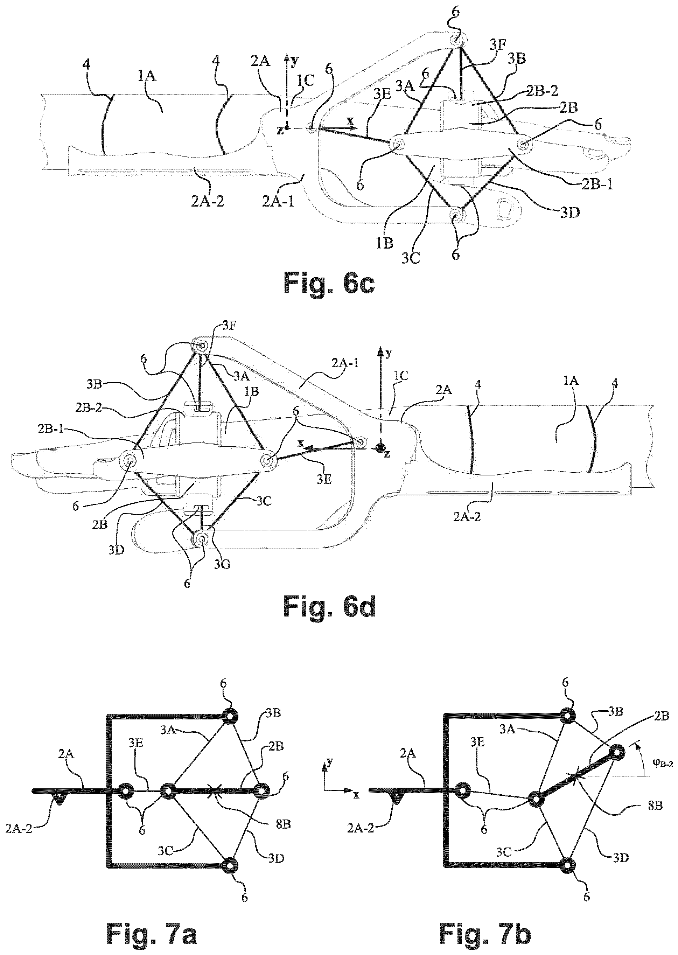

Fig. 6a, Fig. 6b, Fig. 6c, and Fig. 6d show a further embodiment of the orthosis 100.

According to this embodiment, the first fixation element 2B is formed to be fixed to a hand 1B. The first fixation element 2B is composed of segments 2B-1, 2B-2 which are individually 3D-printed. The segment 2B-2 is shaped to abut the fingers of the hand 1B and to fix the first fixation element 2B to the hand 1B.

The rest of the orthosis 102 is formed by a second fixation element 2A comprising the segments 2A-1, 2A-2. Segment 2A-2 serves as a second body abut element abutting a forearm 1A. A body fixation element 4 in the shape of a band or a flexible tape 4 fixes the second body abut element 2A-2 to the forearm 1A.

In the depicted embodiment, one of the fixation elements 2A, 2B is fixed to the human body 1A, 1B via a form-fit, and the other 2A is fixed to the human body 1A, 1B via a form-fit and a force-fit. Alternatively, form-fittings or form- and force-fittings may be applied for either of the fixation elements.

The orthosis 100 of Fig. 6a, Fig. 6b, Fig. 6c, and Fig. 6d comprises three connecting systems.

The first connecting system is formed by the linear retaining elements 3A, 3B, 3C, 3D, 3E on the right-hand side of the segment 2B-2. It is similar to the connecting system of the orthosis 100 according to the embodiment of Fig. 5b, Fig. 5c, and Fig. 5d.

The third connecting system is formed by the linear retaining elements 3A, 3B, 3C, 3D, 3E on the left-hand side of the segment 2B-2. It is similar to the connecting system of the orthosis 100 according to the embodiment of Fig. 5b, Fig. 5c, and Fig. 5d.

Correspondingly, the orthosis 100 can be adapted to any joint of the human body by arranging the first and third connecting system within the orthosis 100 such that the resulting orthosis 100 fits the respective human body part with the first and third connecting systems arranged on opposite sides of the body part.

The first and third connecting systems primarily define the stiffness of the orthosis 100 against a rotation of the first fixation element 2B around the z-axis as well as its translatory movement in the x-y-plane.

The second connecting system is formed by the linear retaining elements 3F, 3G. It is similar to the connecting system of the orthosis 100 according to the embodiments of Fig. 1, Fig. 4.

The second connecting system primarily defines a stiffness of the first fixation element 2B against a rotation around the x- and y-axes as well as a translatory relative movement between the first fixation element 2B and the second fixation element 2A in the y-z-plane.

The combination of the three connecting systems permits to adjust the restoring forces onto the hand 1B for each of its degrees of freedom, i.e., for its three rotational degrees of freedom.

The orthosis 100 is in a statically stable state of equilibrium even without being connected to the body regions. In other words, the linear retaining elements 3A, 3B, 3C, 3D, 3E, 3F, 3G have been selected to ensure that no loads are transferred onto the wrist 1C by the orthosis 100.

Alternatively, the linear retaining elements 3A, 3B, 3C, 3D, 3E, 3F, 3G can be selected to exert a preselected force onto the hand 1B when it is in its physiologically neutral position.

At least one linear retaining element of any of the connecting systems can be replaced by a rigid connection element 118 to implement a (partially) static orthosis 100. For example, only rotational mobility about the z-axis is possible if the linear retaining elements 3F, 3G are replaced by rigid connection elements 118. According to another example, only rotational mobility about the x- and y-axes is possible if the linear retaining elements 3A, 3B, 3C, 3D, 3F are replaced by rigid connection elements 118.

As laid out above in the context of the previous embodiments, the linear retaining elements 3A, 3B, 3C, 3D, 3E, 3F, 3G can be exchanged during therapy and the orthosis 100 can be adapted according to the requirements of therapy. Thus, in individual phases of therapy, the relative mobility between the hand 1B and the forearm 1A along predefined directions can be permitted or restrained according to the therapeutic requirements. Furthermore, the stiffness of the orthosis 100 can be modified according to therapeutic requirements by exchanging the linear retaining elements 3A, 3B, 3C, 3D, 3E, 3F, 3G.

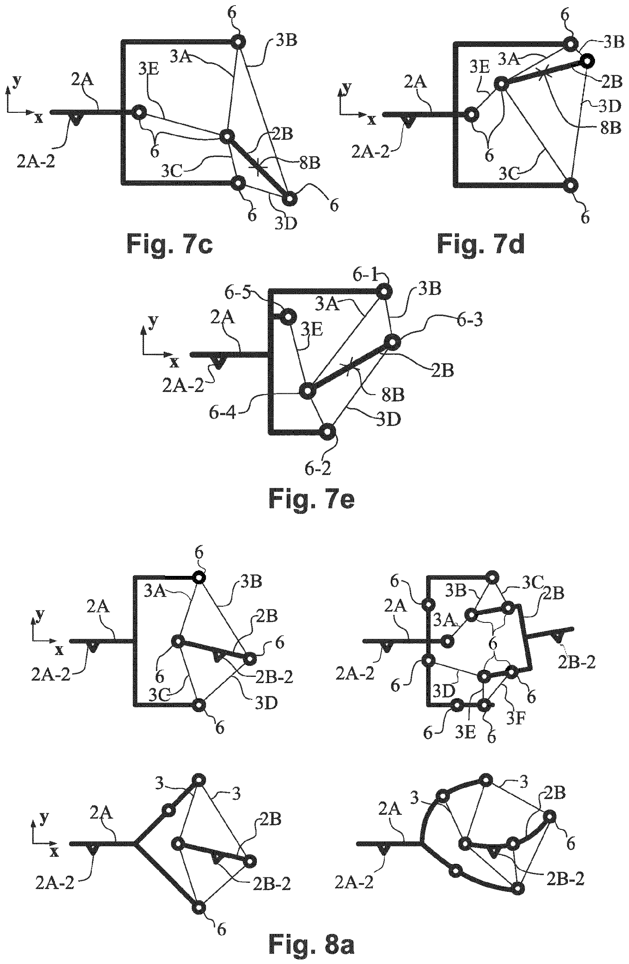

Fig. 7a, Fig. 7b, Fig. 7c, Fig. 7d, and Fig. 7e depict alternative embodiments of the orthosis 100. The depicted embodiments are similar to the one of Fig. 6a, Fig. 6b, Fig. 6c, and Fig. 6d. More specifically, Fig. 7a, Fig. 7b, Fig. 7c, Fig. 7d, and Fig. 7e depict side views of orthoses 100 with different first connecting systems (the third connecting system is formed accordingly), whereas the other elements of the respective orthoses 100 are similar to the corresponding elements of the orthoses 100 of Fig. 6a, Fig. 6b, Fig. 6c, and Fig. 6d.

The orthoses of Fig. 7a, Fig. 7b, Fig. 7c, Fig. 7d, and Fig. 7e each contain five linear retaining elements 3A, 3B, 3C, 3D, 3E connecting the first fixation element 2B to the second fixation element 2A forming the rest of the orthosis 102. The lengths of the linear retaining elements 3A, 3B, 3C, 3D, 3E are smaller in the unassembled state than in the depicted assembled state. As a result, the linear retaining elements 3A, 3B, 3C, 3D, 3E are mechanically biased with a tensile stress. The ends of the linear retaining elements 3A, 3B, 3C, 3D, 3E are attached to the fixation elements 2A, 2B so that they can rotate freely at the attachment elements 6. Due to the elasticity of the linear retaining elements 3A, 3B, 3C, 3D, 3E, they are movable relative to each other in the x-y plane. The relative position of the linear retaining elements 3A, 3B, 3C, 3D, 3E to each other is determined by the mechanical properties, undeformed length and stiffness, of the linear retaining elements 3A, 3B, 3C, 3D, 3E.

In the embodiment of Fig. 7a, the linear retaining elements 3A, 3B, 3C, 3D are identical in their mechanical properties. The first fixation element 2A is symmetrical with respect to the x-axis. For this case, in static equilibrium, the center 8B of the second fixation element 2B is located on the x-axis, and the longitudinal axis of this fixation element 2B coincides with the x-axis, if the influence of weight forces can be neglected. The position of the x-coordinate of the center 8B is defined by the stiffnesses as well as by the undeformed lengths of the linear retaining elements 3A, 3B, 3C, 3D in relation to the stiffness and to the undeformed length of the linear retaining element 3E, respectively, and can be adjusted by suitable selection of these quantities.

In the embodiment of Fig. 7b, the linear retaining elements 3A, 3B have identical mechanical properties. The mechanical properties of the linear retaining elements 3C, 3D are identical, but are different from the mechanical properties of the linear retaining elements 3A, 3B. The first fixation element 2A is symmetrical to the x-axis. For this case, in static equilibrium, the y-coordinate of the center 8B of the second fixation element 2B is non-zero. Whether the center 8B is located at a higher or lower position than in Fig. 7a is determined by the stiffness and by the undeformed length of the linear retaining elements 3A, 3B in relation to the stiffness and to the undeformed length of the linear retaining elements 3C, 3D, respectively, and can be adjusted by suitable selection of these quantities. The position of the x-coordinate of the center 8B as well as the angular position of this segment described by the angle ϕ_{B-2} are defined by the stiffnesses and by the undeformed lengths of the linear retaining elements 3A, 3B, 3C, 3D in relation to the stiffness and to the undeformed length of the linear retaining element 3E, respectively, and can be adjusted by suitable selection of these variables.

According to the embodiments of Fig. 7c, Fig. 7d, the position of the second fixation element 2B in the static stable equilibrium state can be adjusted in a wide range by suitable parameter selection of the linear retaining elements 3A, 3B, 3C, 3D. This can alternatively be achieved by a suitable selection of the geometry of the first fixation element 2A, e.g., by varying the positions of the attachment elements 6-1, 6-2, 6-3, 6-4, 6-5, see Fig. 7e.

According to the embodiments of Fig. 7a, Fig. 7b, Fig. 7c, Fig. 7d, and Fig. 7e, the second fixation element 2B in the equilibrium state is subject to a compressive load. The forces exerted onto the second fixation element 2B at its two ends are equal in magnitude and are directed in opposite directions. Their directions point toward the center 8B. This results in constraints to the position of the second fixation element 2B relative to the rest of the orthosis 102 in equilibrium. For example, the y-coordinates of the attachment elements 6-3, 6-4 cannot both be larger than the y-coordinate of the attachment element 6-1. Moreover, they cannot both be smaller than the y-coordinate of the attachment element 6-2. Also, for example, the x-coordinates of the attachment elements 6-3, 6-4 cannot both be larger than the larger x-coordinate of the attachment elements 6-1, 6-2, nor can the x-coordinates of the attachment elements 6-3, 6-4 be smaller than the smaller x-coordinate of the attachment elements 6-1, 6-2.

The selected linear retaining elements 3A, 3B, 3C, 3D, 3E determine the stiffness of the orthosis 100. This stiffness depends on the direction and on the mechanical bias of the linear retaining elements 3A, 3B, 3C, 3D, 3E. The structure exhibits in each case a given global stiffness during a relative translatory or rotatory relative movement of the fixation element 2B. This stiffness also depends on the magnitude of the relative translation or relative rotation and can be adjusted in a wide range with a suitable choice of the mechanical properties of the linear retaining elements 3A, 3B, 3C, 3D, 3E. This can be achieved without changing the shape of the fixation elements 2A, 2B by selecting linear retaining elements 3A, 3B, 3C, 3D, 3E of proper stiffnesses and thereby changing the mechanical bias of the structure.

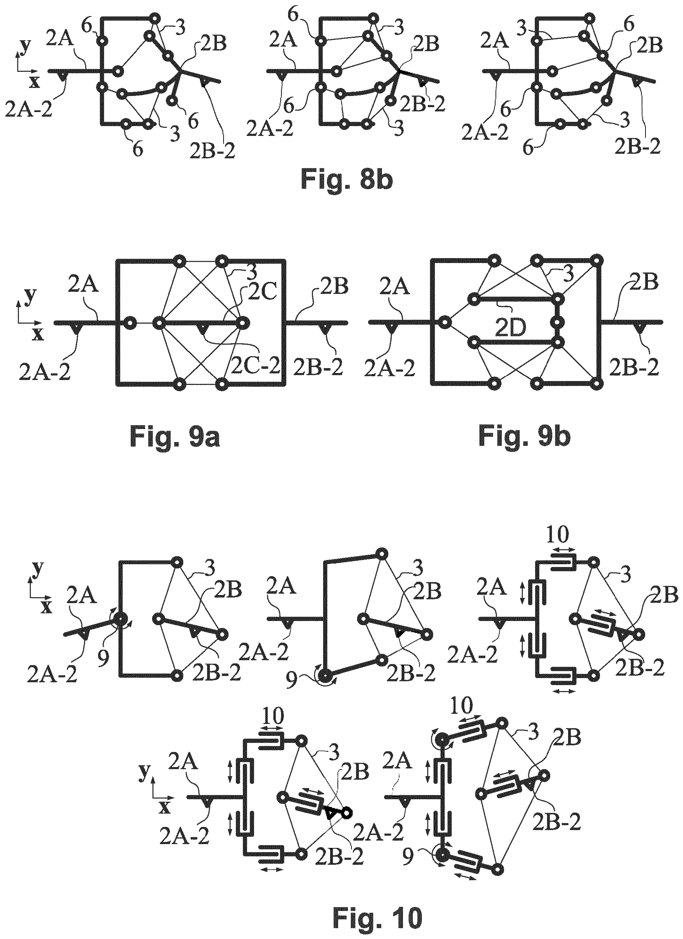

Fig. 8a, Fig. 8b depict alternative embodiments of the orthosis 100. The depicted embodiments are similar to the one of Fig. 6a, Fig. 6b, Fig. 6c, and Fig. 6d. More specifically, Fig. 8a, Fig. 8b depict side views of orthoses 100 with different first connecting systems (the third connecting system is formed accordingly), whereas the other elements of the respective orthoses are similar to the corresponding elements of the orthoses 100 of Fig. 6a, Fig. 6b, Fig. 6c, and Fig. 6d.

The orthoses 100 according to the embodiments of Fig. 8a, Fig. 8b comprise additional attachment elements 6. In each of the depicted embodiments, at least two attachment elements 6 are provided per fixation element 2A, 2B to ensure static stability of the equilibrium position of the structure.

In the design of the orthosis 100, collision between the fixation elements 2A, 2B or between one of the fixation elements 2A, 2B and the human body can be prevented by properly shaping the individual fixation elements 2A, 2B. The directional stiffness of the structure can be influenced by providing additional attachment elements 6 and attaching additional linear retaining elements at the additional attachment elements 6. Fig. 8b shows embodiments of orthoses 100 with different numbers of linear retaining elements.

Fig. 9a, Fig. 9b depict alternative embodiments of the orthosis 100. The depicted embodiments are similar to the one of Fig. 6a, Fig. 6b, Fig. 6c, and Fig. 6d. More specifically, Fig. 9a, Fig. 9b depict side views of orthoses 100 with different first connecting systems (the third connecting system is formed accordingly), whereas the other elements of the respective orthoses are similar to the corresponding elements of the orthoses 100 of Fig. 6a, Fig. 6b, Fig. 6c, and Fig. 6d.

The orthoses 100 of Fig. 9a, Fig. 9b have more than two fixation elements 2A, 2B, 2C or more than two positions 2A-2, 2B-2, 2C-2 at which they are fixed to the human body.

In the embodiment of Fig. 9b, the third fixation element 2C is not fixed to the human body directly, but only via the fixation elements 2A, 2B. The third fixation element 2C is referred to as a suspended fixation element 2C.

Orthoses 100 with more than two fixation elements are beneficial for connecting two body regions whose relative mobility to each other is to be influenced at one point. In these embodiments, there is at least one fixation element that is not directly connected to a body region (suspended fixation element). The suspended fixation element is connected to the first and second fixation elements 2A, 2B by linear retaining elements, respectively. The direction-dependent stiffness of the structure can be adjusted by the selection of the linear retaining elements.

Fig. 10 shows orthoses 100 according to various embodiments with hinged connections. Each hinged connection is adapted to provide a rotational adjustment 9 or a translational adjustment 10 between a respective linking element and a respective fixation element the linking element is connected to. The hinged connections are implemented as swivel and/or push joints.

These embodiments enhance the possibility of reversibly changing the shape of the orthosis 100, with the following advantages:
- improved adaptation of the orthosis 100 to the anatomical conditions of the affected body regions,
- improved adjustment of the orthosis 100 according to therapeutic specifications for the relative position of the affected body regions,
- on-the-fly adjustment of the mechanical bias of the linear retaining elements and thus of the restoring forces of the second fixation elements 2B with respect to its various degrees of freedom.

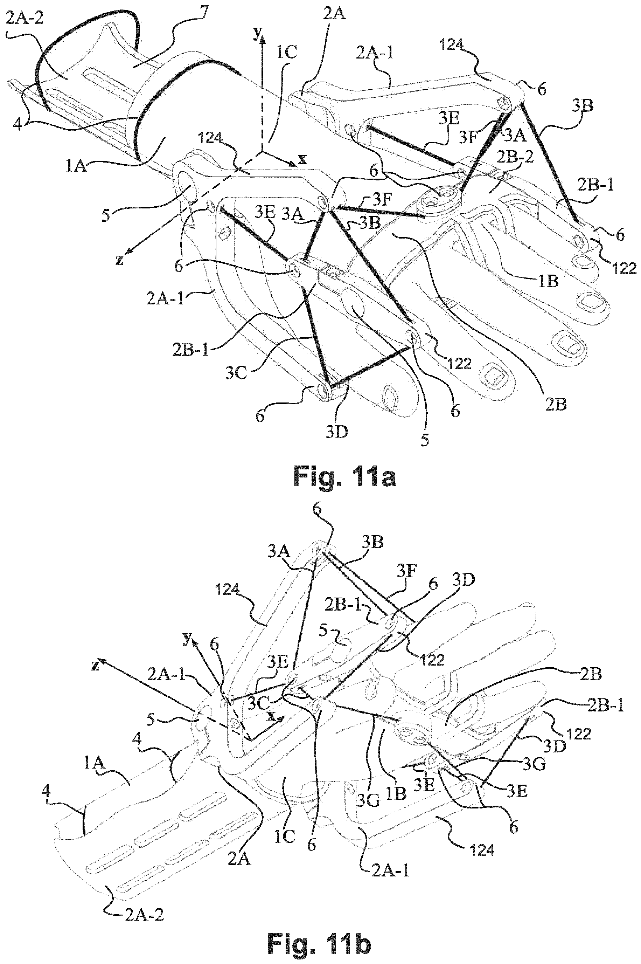

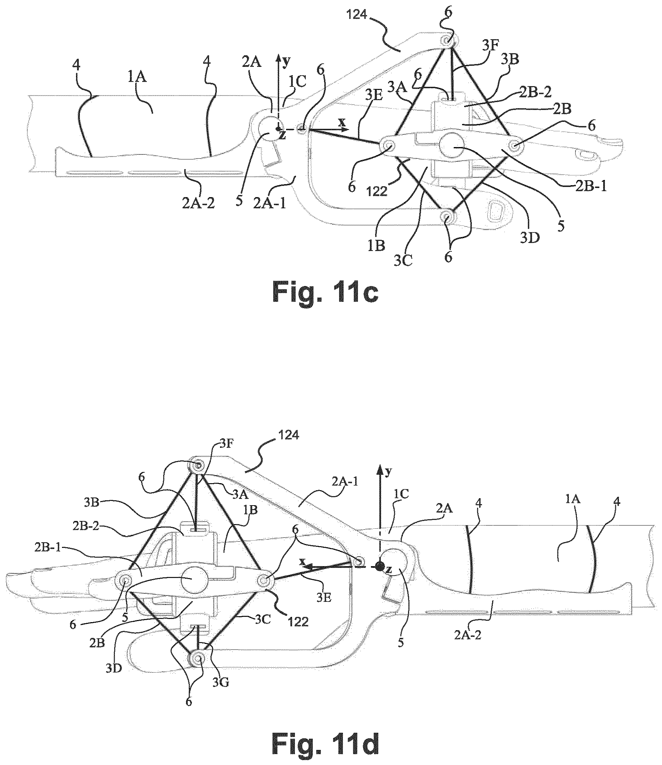

Fig. 11a, Fig. 11b, Fig. 11c, and Fig. 11d depict an alternative embodiment of the orthosis 100. The depicted embodiment is similar to the one of Fig. 6a, Fig. 6b, Fig. 6c, and Fig. 6d.

In this embodiment, the orthosis 100 comprises linking elements 122, 124 to provide the advantages laid out above in the context of the linking elements of the other embodiments. The orthosis 100 comprises adjustable connections 5 between the linking elements 122, 124 and the respective first or second fixation element 2A, 2B that the respective linking element 122, 124 is connected to. The linear retaining elements 3A, 3B, 3C, 3D, 3E, 3F, 3G are attached to the first and second fixation elements 2A, 2B via the linking elements 122, 124. More specifically, at least three of the linear retaining elements 3A, 3B, 3C, 3D, 3E, 3F, 3G are attached to any of the linking elements 122, 124. Any of the linear retaining elements 3A, 3B, 3C, 3D, 3E, 3F, 3G is connected to a linking element 122, 124 at either of its ends.

### LIST OF REFERENCE SIGNS

- 100: orthosis
- 2B: first fixation element
- 3A, 3C: first, second linear retaining element
- 3B, 3D, 3E: third, fourth linear retaining element
- 102: rest of orthosis
- 103A, 103C: first, second force
- 103B, 103D, 103E: third, fourth force
- y: first orientation
- +y, -y: directions along the first orientation
- x: second orientation
- +x, -x: directions along the second orientation
- z: third orientation
- 6, 6-1, 6-2, 6-3, 6-4, 6-5: attachment elements
- 6': attachment points
- 108: bolt, screw
- 110a, 110b: retainment elements of different stiffnesses
- 112: retainment element with multiple lugs
- 114: retainment element with multiple hooks
- 116: length limiter
- 118: rigid connection element
- 130: second adjustment element
- 122, 124, 126, 128: linking elements
- 10: translational adjustment
- 120: integral piece combining multiple linear retaining elements
- 1A: first position of human body, forearm
- 1B: second position of human body, hand
- 1C: joint of human body, wrist
- 2B-1, 2B-2: segments of first fixation element
- 2A: second fixation element
- 2A-1, 2A-2: segments of second fixation element
- 4: body fixation element
- 7: cushioning
- 8B: position/center of first fixation element
- 2C: third fixation element
- 2C-1, 2C-2: segments of third fixation element
- 2D: suspended element
- 9: rotational adjustment

## Claims

1. Orthosis (100), wherein the orthosis (100) is adapted to be fixed to a human body at a fixing position, the orthosis (100) comprising:
a first fixation element (2B) for being fixed to the human body at a first position different from the fixing position;
a first connecting system, wherein the first connecting system is adapted to enable a translationally and/or rotationally movable connection of the first fixation element (2B) to the orthosis (100); the first connecting system comprising
a first linear retaining element (3A), a second linear retaining element (3C), and a third linear retaining element (3E), each for providing a force along a direction along which the respective linear retaining element extends;
wherein the orthosis (100) is adapted for attaching each of the first, second, and third linear retaining element (3A, 3C, 3E) to the first fixation element (2B) and to a rest of the orthosis (102), such that the first, second, and third linear retaining element (3A, 3C, 3E) exerts a respective first, second, and third force (103A, 103C, 103E) onto the first fixation element (2B);
wherein the first force (103A) and the second force (103C) comprise respective components along a first direction (y) with opposite orientations (+y, -y), and comprise respective components along a second direction (x) with a same, first orientation (+x), and
wherein the third force (103E) comprises a component along the second direction (x) with a second orientation (-x) opposite to the first orientation (+x).

2. Orthosis (100) according to claim 1,
wherein:
each of the first, second, and third linear retaining element (3A, 3C, 3E) is an elastic element adapted to be mechanically biased with a tensile stress when attached to the first fixation element (2B) and to the rest of the orthosis (102); wherein, optionally, the elastic element comprises or is made of a polymeric elastomer;
and/or:
wherein the first connecting system is arranged in a plane when attached to the orthosis (100);
and/or:
wherein the first, second, and third linear retaining element (3A, 3C, 3E) are arranged in a plane when attached to the orthosis (100).

3. Orthosis (100) according to claim 1 or 2, further comprising:
a linking element (122, 124, 126, 128), wherein the orthosis (100) is adapted for attaching at least one of the first, second, and third linear retaining element (3A, 3C, 3E) to the first fixation element (2B) or to the rest of the orthosis (102) via the linking element (122, 124, 126, 128), and
an adjustable connection between the linking element and the first fixation element (2B) or to the rest of the orthosis (102) for adjusting a length or an orientation of said linear retaining element,
wherein the adjustable connection is adapted to provide a locked state wherein a position of the linking element relative to the first fixation element (2B) or to the rest of the orthosis (102), that the linking element is connected to, is fixed, and an unlocked state wherein said position is moveable;
wherein optionally:
at least one or at least two or all three of the first linear retaining element (3A), the second linear retaining element (3C), and the third linear retaining element (3E) is/are attached to the first fixation element (2B) or to the rest of the orthosis (102) via the linking element.

4. Orthosis (100) according to claim 3,
wherein, according to a first alternative:
the orthosis (100) comprises:
a first linking element with the features of the linking element of claim 3, and
a second linking element with the features of the linking element of claim 3, wherein the adjustable connection of the first linking element is between the first linking element and the first fixation element (2B),
wherein the adjustable connection of the second linking element is between the second linking element and the rest of the orthosis (102), and
wherein the same retaining element is or the same retaining elements are attached via the first linking element to the first fixation element (2B) and via the second linking element to the rest of the orthosis (102);
and/or wherein, according to a second alternative:
at least one or all of the adjustable connections of the linking element, the first linking element, and the second linking element is a hinged connection adapted to provide a rotational adjustment or a translational adjustment between the respective linking element and the element it is connected to.

5. Orthosis (100) according to any of the preceding claims,
wherein:
the first fixation element (2B) comprises:
a first body abut element, wherein the first body abut element is adapted to abut a first section of the human body, and
a first body fixation element, wherein the first body fixation element is adapted to fix the first body abut element to the human body,
wherein the first body abut element is mechanically rigid;
and/or:
wherein the rest of the orthosis (102) comprises:
a second body abut element, wherein the second body abut element is adapted to abut a second section of the human body, and
a second body fixation element, wherein the second body fixation element is adapted to fix the second body abut element to the human body,
wherein the second body abut element is mechanically rigid;
and wherein, optionally:
the rest of the orthosis (102) comprises a second fixation element (2A, 2C),
wherein the second fixation element (2A, 2C) comprises the second body abut element and the second body fixation element.

6. Orthosis (100) according to any of the preceding claims, wherein the first connecting system further comprises a fourth linear retaining element (3B, 3D), for providing a force along a direction along which the fourth linear retaining element (3B, 3D) extends; wherein the orthosis (100) is adapted for attaching the fourth linear retaining element to the first fixation element (2B) and to the rest of the orthosis (102), such that the fourth linear retaining element (3B, 3D) exerts a fourth force (103B, 103D) onto the first fixation element (2B);
wherein the fourth force comprises a component along the second direction (x) along the second orientation (-x), and
wherein the first, second, third, and fourth force result in a compressive load onto the first fixation element (2B),
wherein, optionally, the first, second, third, and fourth linear retaining element (3A, 3B, 3C, 3D, 3E) are arranged in a plane when attached to the orthosis (100).

7. Orthosis (100) according to any of the preceding claims, wherein the first fixation element (2B) and the rest of the orthosis (102) comprise attachment elements (6) for attaching the first, second, and third linear retaining element (3A, 3C, 3E) to the first fixation element (2B) and to the rest (102) of the orthosis, and
wherein the attachment elements (6) are located on the first fixation element (2B) and on the rest of the orthosis (102) to determine directions of the first, second, and third force (103A, 103C, 103E);
wherein optionally, according to a first alternative:
the attachment elements (6) or at least one of the attachment elements (6) comprise(s) a first adjustment element adapted for adjusting an orientation and/or a mechanical bias of the first, second, or third linear retaining element (3A, 3C, 3E) when attached to the first fixation element (2B) and to the rest of the orthosis (102);
and/or, wherein optionally, according to a second alternative:
the first adjustment element provides a plurality of attachment points (6') on the first fixation element (2B) or on the rest (102) of the orthosis for the first, second, or third linear retaining element (3A, 3C, 3E);
and/or, wherein optionally, according to a third alternative:
the first, second, or third linear retaining element (3A, 3C, 3E) or at least one of the first, second, or third linear retaining element (3A, 3C, 3E) comprises a second adjustment element for adjusting a mechanical bias of the first, second, or third linear retaining element (3A, 3C, 3E) when attached to the first fixation element (2B) and to the rest of the orthosis (102).

8. Orthosis (100) according to any of the preceding claims,
wherein:
the second adjustment element provides a plurality of attachment points for attaching the respective linear retaining element to the first fixation element (2B) or to the rest (102) of the orthosis;
and/or:
wherein the orthosis (100) further comprises:
at least one additional linear retaining element,
wherein the at least one additional linear retaining element is adapted to be attached to the first fixation element (2B) and to the rest of the orthosis (102) in place of the first, second, or third linear retaining element (3A, 3C, 3E), and wherein the at least one additional linear retaining element is **characterized by** a stiffness exceeding a stiffness of said linear retaining element (3A, 3C, 3E) to be replaced or exceeding the stiffness of each of the first, second, or third linear retaining element (3A, 3C, 3E);
and/or:
wherein at least two or all of the first, second, or third linear retaining element (3A, 3C, 3E) are formed as an integral piece;
and/or:
wherein the orthosis (100) further comprises at least one length limiter adapted to limit a maximum length of at least one or all of the first, second, and third linear retaining element (3A, 3C, 3E), wherein, optionally, the at least one length limiter is adapted to be attached to the first fixation element (2B) and to the rest of the orthosis (102) in place of or parallel to the first, second, or third linear retaining element (3A, 3C, 3E) and/or
wherein, optionally, the at least one length limiter comprises a wire or a string adapted to be attached to the first fixation element (2B) and to the rest of the orthosis (102) in place of or parallel to the first, second, or third linear retaining element (3A, 3C, 3E);
and/or:
wherein the orthosis (100) further comprises:
at least one rigid connection element,
wherein the orthosis (100) is adapted for attaching the at least one rigid connection element to the first fixation element (2B) and to the rest of the orthosis (102) in place of the first, second, or third linear retaining element (3A, 3C, 3E) to suppress at least one degree of freedom of the translationally and/or rotationally movable connection of the first fixation element (2B) to the orthosis (100).

9. Orthosis (100) according to any of the preceding claims, which further comprises a second connecting system with the features of the first connecting system according to claim 1,
wherein the first connecting system is adapted to adjust a first restoring force of a translationally movable connection of the first fixation element (2B) to the orthosis (100) in a first plane, and the second connecting system is adapted to adjust a second restoring force of a translationally movable connection of the first fixation element (2B) to the orthosis (100) in a second plane perpendicular to the first plane, and/or
wherein the first connecting system is adapted to adjust a third restoring force of a rotationally movable connection of the first fixation element (2B) to the orthosis (100) around a first axis of rotation, and the second connecting system is adapted to adjust a fourth restoring force of a rotationally movable connection of the first fixation element (2B) to the orthosis (100) around a second axis of rotation perpendicular to the first axis of rotation.

10. Orthosis (100) according to claim 9,
wherein:
the first connecting system is adapted to adjust the third restoring force, and the second connecting system is adapted to adjust the fourth restoring force, and wherein the orthosis (100) further comprises a third connecting system with the features of the first connecting system according to claim 1, wherein the third connecting system is adapted to adjust the third restoring force together with the first connecting system, and wherein
the first connecting system and the third connecting system are arranged on opposite sides of the first position;
and/or:
wherein the first connecting system is adapted to enable a translational movement of the first fixation element (2B) in a plane parallel to a plane in which the first connecting system extends when connected to the orthosis;
and/or:
wherein the first connecting system and the second connecting system are oriented perpendicular to each other when connected to the orthosis.

11. Orthosis (100) according to any of the preceding claims,
wherein the orthosis, in particular the first fixation element (2B) and/or any other fixation element of the orthosis (100), is provided with a cushioning towards the human body;
and/or:
wherein the orthosis is a hand orthosis, a wrist orthosis, a finger orthosis, a shoulder orthosis, or an ankle orthosis;
and/or:
wherein the first fixation element (2B) and/or any other fixation element of the orthosis (100) and/or the rest of the orthosis (102) is adapted to provide a form-fit and/or a force-fit connection to the human body; in particular, wherein the form-fit and/or the force-fit connection to the human body is based on a measured anatomy of a human body.

12. Method for adjusting an orthosis (100), wherein the orthosis (100) is adapted to be fixed to a human body at a fixing position, the orthosis (100) comprising:
a first fixation element (2B) for being fixed to the human body at a first position different from the fixing position;
a first connecting system, wherein the first connecting system is adapted to enable a translationally and/or rotationally movable connection of the first fixation element (2B) to the orthosis (100); the first connecting system comprising
a first linear retaining element (3A), a second linear retaining element (3C), and a third linear retaining element (3E), each for providing a force along a direction along which the respective linear retaining element extends;
wherein the first, second, and third linear retaining element (3A, 3C, 3E) is attached to the first fixation element (2B) and to a rest of the orthosis (102), such that the first, second, and third linear retaining element (3A, 3C, 3E) exerts a respective first, second, and third force (103A, 103C, 103E) onto the first fixation element (2B);
wherein the first force (103A) and the second force (103C) comprise respective components along a first direction (y) with opposite orientations (+y, -y), and comprise respective components along a second direction (x) with a same, first orientation (+x), and
wherein the third force (103E) comprises a component along the second direction (x) with a second orientation (-x) opposite to the first orientation (+x),
the method comprising:
adjusting an orientation or a mechanical bias of at least one of the first, second, and third linear retaining element (3A, 3C, 3E).

13. The method of claim 12,
wherein:
the orientation and/or the mechanical bias of the at least one of the first, second, and third linear retaining element (3A, 3C, 3E) is adjusted such that in a physiologically neutral position of a part of the human body located between the fixing position and the first position, no forces result onto the fixing position and/or onto the first position when the orthosis (100) is fixed to the human body;
or:
wherein the orientation and/or the mechanical bias of the at least one of the first, second, and third linear retaining element (3A, 3C, 3E) is adjusted such that in a physiologically neutral position of a part of the human body located between the fixing position and the first position, a preselected force results onto the first position and/or onto the first fixation element (2B) when the orthosis (100) is fixed to the human body.

14. The method of claim 12 or 13, wherein the orientation and/or the mechanical bias of the at least one of the first, second, and third linear retaining element (3A, 3C, 3E) is adjusted such that, when a joint of a human body located between the fixing position and the first position is rotated, no forces result onto the joint.

15. The method of any of claims 12-14,
wherein:
the adjusting the orientation and/or the mechanical bias of at least one of the first, second, and third linear retaining element (3A, 3C, 3E) comprises:
replacing at least one of the first, second, and third linear retaining element (3A, 3C, 3E) with at least one additional linear retaining element,
wherein the at least one additional linear retaining element is adapted to provide a stiffness exceeding a stiffness of said linear retaining element (3A, 3C, 3E) to be replaced or exceeding the stiffness of each of the first, second, or third linear retaining element (3A, 3C, 3E);
and/or:
wherein the first fixation element (2B) and the rest of the orthosis (102) comprise attachment elements (6) for attaching the first, second, and third linear retaining element (3A, 3C, 3E) to the first fixation element (2B) and to the rest of the orthosis (102),
wherein the attachment elements (6) are located on the first fixation element (2B) and on the rest of the orthosis (102) to determine directions of the first, second, and third force (103A, 103C, 103E),
wherein the attachment elements (6) or at least one of the attachment elements (6) comprises a first adjustment element adapted for adjusting an orientation and/or a mechanical bias of the first, second, or third linear retaining element (3A, 3C, 3E) when the respective linear retaining element (3A, 3C, 3E) is attached to the first fixation element (2B) and to the rest of the orthosis (102), and
wherein the adjusting the orientation or the mechanical bias of at least one of the first, second, and third linear retaining element (3A, 3C, 3E) comprises changing the attachment of the at least one of the first, second, and third linear retaining element (3A, 3C, 3E) to the respective attachment element (6) using the first adjustment element;
and/or:
wherein the first, second, or third linear retaining elements (3A, 3C, 3E) or at least one of the first, second, or third linear retaining element (3A, 3C, 3E) comprises a second adjustment element,
wherein the adjusting the orientation or the mechanical bias of at least one of the first, second, and third linear retaining element (3A, 3C, 3E) comprises changing the attachment of the at least one of the first, second, and third linear retaining element (3A, 3C, 3E) to the first fixation element (2B) or to the rest of the orthosis (102) using the second adjustment element;
and/or:
wherein the orthosis further comprises:
a linking element, wherein at least one of the first linear retaining element (3A), the second linear retaining element (3C), and the third linear retaining element (3E) is attached to the first fixation element (2B) or to the rest of the orthosis (102) via the linking element, and
an adjustable connection between the linking element and the first fixation element (2B) or to the rest of the orthosis (102), and
wherein the adjusting the orientation or the mechanical bias of at least one of the first, second, and third linear retaining element (3A, 3C, 3E) comprises adjusting a position of the linking element;
wherein, optionally:
the adjustable connection is adapted to provide a locked state wherein a position of the linking element relative to the first fixation element (2B) or to the rest of the orthosis (102) that the linking element is connected to is fixed, and an unlocked state wherein said position is moveable, and wherein the adjusting the position of the linking element is performed while the adjustable connection is in the unlocked state, and
wherein the method further comprises setting the adjustable connection to the locked state.
